(19) Europäisches Patentamt
European Patent Office
Office européen des brevets

(11) EP 0 801 947 B1

(12) **FASCICULE DE BREVET EUROPEEN**

(45) Date de publication et mention de la délivrance du brevet:
**18.11.1998 Bulletin 1998/47**

(51) Int. Cl.$^6$: **A61K 7/48**

(21) Numéro de dépôt: **97400340.2**

(22) Date de dépôt: **14.02.1997**

(54) **Ultilisation de l'octopirox comme agent depigmentant**

Verwendung von Octopirox als Hautdepigmentierungsmittel

Use of octopirox as skin depigmentation agent

(84) Etats contractants désignés:
**DE ES FR GB IT**

(30) Priorité: **18.03.1996 FR 9603343**

(43) Date de publication de la demande:
**22.10.1997 Bulletin 1997/43**

(73) Titulaire: **L'OREAL**
**75008 Paris (FR)**

(72) Inventeur: **Fanchon, Chantal**
**75015 Paris (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**EP-A- 0 496 433** **WO-A-95/27485**

- **PATENT ABSTRACTS OF JAPAN vol. 12, no. 455 (C-548) & JP 63 179813 A (EIKOUDOU KK)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.6/3.4

## Description

La présente invention se rapporte à l'utilisation de l'octopirox comme agent dépigmentant ou blanchissant dans une composition cosmétique et/ou dermatologique, ainsi qu'à une composition dépigmentante contenant de l'octopirox.

La couleur de la peau humaine est fonction de différents facteurs et notamment des saisons de l'année, de la race et du sexe, et elle est principalement déterminée par la concentration et la nature de la mélanine produite par les mélanocytes. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kéranocytes situés à la surface de la peau.

Le mécanisme de formation de la pigmentation de la peau, c'est-à-dire de la formation de la mélanine est particulièrement complexe et fait intervenir schématiquement les principales étapes suivantes :

Tyrosine ---> Dopa ---> Dopaquinone ---> Dopachrome ---> Mélanine

La tyrosinase est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de la toxicité qu'ils entraînent, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi l'hydroquinone est un composé particulièrement irritant et cytotoxique pour le mélanocyte, dont le remplacement, total ou partiel a été envisagé par de nombreux auteurs.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo. Pour ces dernières, à défaut de pouvoir repigmenter la peau lésée, on achève de dépigmenter les zones de peau normale résiduelle pour donner à l'ensemble de la peau une teinte blanche homogène.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique. Malheureusement, ce composé est instable en solution, ce qui complique quelque peu la fabrication de la composition.

Aussi, il subsiste le besoin d'un nouvel agent blanchissant de la peau humaine à action aussi efficace que ceux connus mais n'ayant pas leurs inconvénients, c'est-à-dire qui soit stable dans une composition et non toxique pour la peau.

La demanderesse a trouvé de manière inattendue que l'octopirox présentait la propriété d'inhiber l'activation de la tyrosinase, et donc la synthèse de la mélanine, et pouvait agir ainsi sur la pigmentation et les taches de la peau sans toxicité.

L'octopirox est la 1-hydroxy-4-méthyl-6-(2,4,4-triméthylpentyl)-2(1H)-pyridinone de formule :

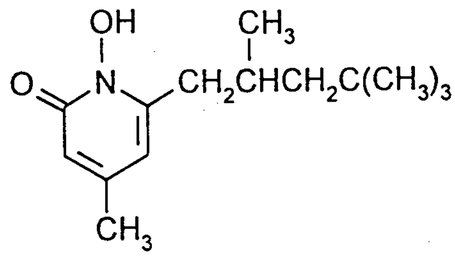

Certes il est connu d'utiliser l'octopirox comme agent antimycotique (voir document FR-A-2022146), comme agent antichute (voir document FR-A-2618068) et comme agent anti-acné (voir document EP-A-218410). Toutefois personne jusqu'à ce jour n'avait envisagé de l'utiliser comme agent dépigmentant.

La présente invention a donc pour objet l'utilisation de l'octopirox dans et/ou pour la fabrication d'une composition cosmétique et/ou dermatologique pour dépigmenter et/ou blanchir la peau humaine et/ou enlever les taches pigmentaires de la peau.

La présente invention a aussi pour objet l'utilisation de l'octopirox pour la fabrication d'une composition cosmétique et/ou dermatologique, comme inhibiteur de la tyrosinase et/ou de la synthèse de la mélanine.

La présente invention a aussi pour objet l'utilisation de l'octopirox pour la fabrication d'une composition cosmétique dépigmentante et/ou blanchissante de la peau humaine.

La présente invention a encore pour objet l'utilisation de l'octopirox pour la fabrication d'une composition dermatologique pour le blanchiment et/ou la dépigmentation de la peau humaine.

La présente invention se rapporte également à un procédé cosmétique et/ou de dépigmentation et/ou de blanchiment de la peau humaine consistant à appliquer sur la peau pigmentée une composition comprenant l'octopirox.

La composition selon l'invention est appropriée pour une utilisation topique et contient donc un milieu cosmétiquement ou dermatologiquement acceptable, c'est-à-dire compatible avec la peau.

La présente invention a aussi pour objet une composition dépigmentante, caractérisée en ce qu'elle contient dans un milieu cosmétiquement et/ou dermatologiquement acceptable, de l'octopirox.

L'octopirox peut être notamment présent en une quantité allant de 0,01 à 10 % et de préférence de 0,3 à 3 % du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. En cas d'incompatibilité, ces actifs et/ou l'octopirox peuvent être incorporés dans des sphérules, notamment des vésicules ioniques ou non-ioniques et/ou des nanoparticules (nanocapsules et/ou nanosphères), de manière à les isoler les uns des autres dans la composition.

**Test:**

Un test a mis en évidence l'activité de l'octopirox comme inhibiteur de la tyrosinase par évaluation de son effet inhibiteur sur l'activité dopa-oxydase de la tyrosinase des mélanocytes de champignons.

Selon ce test, on suit par spectrométrie visible à 475 nm la quantité de dopachrome formée au cours de la chaîne de réaction de transformation de la tyrosine en mélanine, en fonction du temps. Ces réactions sont catalysées in vitro par la tyrosinase de champignons, en présence d'un co-substrat réducteur (la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation de mélanine.

Le milieu réactionnel utilisé pour réaliser ce test comprend 1 ml d'une solution de L-tyrosine à $2.10^{-3}$ M dans du tampon phosphate 0,1 M de pH = 6,5, 0,1 ml de solution de L-dopa à $10^{-4}$ M dans le tampon phosphate, 0,05 ml de solution de tyrosinase de champignons à 2.400 unités/ml dans le tampon phosphate et 1,85 ml de solution de l'inhibiteur à $10^{-2}$ M dans le tampon phosphate. Pour le témoin, la quantité d'inhibiteur a été remplacée par de l'éthanol. Le zéro des courbes a été étalonné à l'aide du tampon phosphate.

La concentration de $10^{-2}$ M d'octopirox dans le milieu réactionnel correspond à une quantité équimolaire par rapport à la tyrosine utilisée.

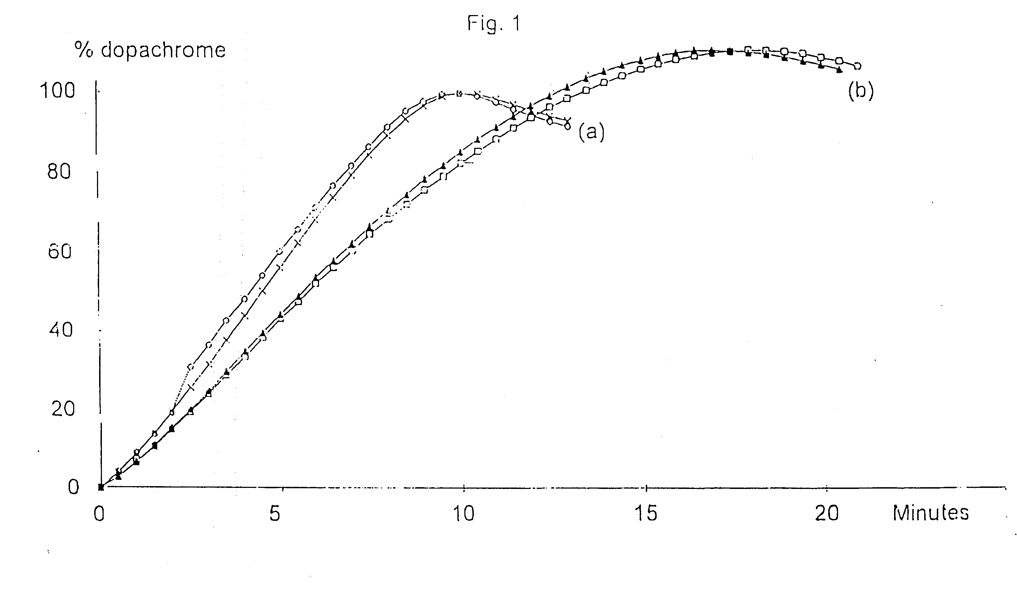

Les courbes présentées sur la figure 1 ont été tracées en suivant, en ordonnée, le pourcentage de dopachrome formée en fonction du temps indiqué en abscisse et exprimé en minutes. Les courbes (a) sont relatives au témoin et les courbes (b) sont relatives à l'octopirox

En comparant ces deux courbes, on voit nettement que la présence d'octopirox entraîne un net ralentissement de la quantité de dopachrome formée au cours du temps, ce qui signifie que l'octopirox présente un effet d'inhibition de la tyrosinase. En particulier, pour obtenir le même pourcentage de dopachrome formée, par exemple 100 %, il faut attendre 14 minutes avec l'octopirox au lieu de 9,5 minutes avec le témoin.

L'invention va maintenant être illustrée à l'aide des exemples qui suivent. Les concentrations sont données en pourcentage en poids.

**Exemple 1 : Crème traitante**

| | |
|---|---|
| - Alcool cétylique | 1,05 % |
| - Stéarate de PEG-20 (Myrj 49 vendu par la société ICI) | 1,65 % |
| - Stéarate de glycéryle | 0,3 % |
| - Cyclométhicone | 6 % |
| - Octopirox | 0,5 % |
| - Propylène glycol | 6 % |
| - Carbomer | 0,6 % |
| - Glycérine | 3 % |

(suite)

| | |
|---|---|
| - Triéthanolamine | 1 % |
| - Conservateurs | 0,5 % |
| - Eau déminéralisée qsp | 100 % |

La crème obtenue utilisée en application quotidienne, permet d'obtenir un blanchiment de la peau.

**Exemple 2 : Gel traitant**

| | |
|---|---|
| - Propylène glycol | 10 % |
| - Alcool éthylique | 40 % |
| - Glycérine | 3 % |
| - Octopirox | 0,5 % |
| - Conservateurs | 0,15 % |
| - Parfum | 0,15 % |
| - Eau déminéralisée qsp | 100 % |

Le gel obtenu peut être utilisé quotidiennement et est apte à dépigmenter la peau.

**Exemple 3 : Stick traitant**

| | |
|---|---|
| - Cire de Carnauba | 5 % |
| - Ozokerite | 7 % |
| - Alcool cétylique | 1,4 % |
| - Lanoline | 6 % |
| - Oxydes de fer (pigments) | 4 % |
| - Dioxyde de titane (pigments) | 20 % |
| - Amidon de riz (charge) | 7 % |
| - Acide salicylique | 1 % |
| - EDTA | 0,1 % |
| - Octopirox | 2 % |
| - Perhydrosqualène qsp | 100 % |

Le stick obtenu, utilisé sur les taches pigmentaires, permet de les atténuer voire de les faire disparaître.

**Revendications**

1. Utilisation de l'octopirox pour la fabrication d'une composition cosmétique ou pour la fabrication d'une composition dermatologique pour dépigmenter et/ou blanchir la peau humaine et/ou enlever les taches pigmentaires de la peau.

2. Utilisation de l'octopirox pour la fabrication d'une composition cosmétique ou pour la fabrication d'une composition dermatologique, comme inhibiteur de la tyrosinase et/ou de la synthèse de la mélanine.

3. Utilisation non thérapeutique de l'octopirox pour la fabrication d'une composition cosmétique dépigmentante et/ou blanchissante de la peau humaine.

4. Utilisation de l'octopirox pour la fabrication d'une composition dermatologique dépigmentante et/ou blanchissante de la peau humaine.

5. Utilisation selon l'une quelconque des revendications précédentes, caractérisée en ce que l'octopirox est présent en une quantité allant de 0,01 à 10 % du poids total de la composition.

6. Utilisation selon l'une quelconque des revendications précédentes, caractérisée en ce que l'octopirox est présent en une quantité allant de 0,3 à 3 % du poids total de la composition.

7. Utilisation selon l'une quelconque des revendications précédentes, caractérisée en ce que la composition comprend en outre, au moins un actif choisi parmi les agents kératolytiques et/ou desquamants, anti-inflammatoires, apaisants et leurs mélanges.

8. Utilisation selon la revendication précédente, caractérisée en ce que l'octopirox et/ou l'actif est encapsulé dans des sphérules.

9. Procédé cosmétique de dépigmentation et/ou blanchiment de la peau humaine en excluant un procédé thérapeutique, caractérisé en ce qu'il consiste à appliquer sur la peau pigmentée une composition comprenant l'octopirox.

## Claims

1. Use of octopirox for the manufacture of a cosmetic composition or for the manufacture of a dermatological composition to depigment and/or bleach human skin and/or to remove skin pigment marks.

2. Use of octopirox for the manufacture of a cosmetic composition or for the manufacture of a dermatological composition, as an inhibitor of tyrosinase and/or of the synthesis of melanin.

3. Non-therapeutic use of octopirox for the manufacture of a cosmetic composition for depigmenting and/or bleaching human skin.

4. Use of octopirox for the manufacture of a dermatological composition for depigmenting and/or bleaching human skin.

5. Use according to any one of the proceding claims, characterized in that the octopirox is present in an amount ranging from 0.01 to 10% of the total weight of the composition.

6. Use according to any one of the preceding claims, characterized in that the octopirox is present in an amount ranging from 0.3 to 3% of the total weight of the composition.

7. Use according to any one of the preceding claims, characterized in that the composition also comprises at least one active agent chosen from keratolytic and/or desquamating agents, anti-inflammatory agents, calmants and mixtures thereof.

8. Use according to the preceding claim, characterized in that the octopirox and/or the active agent is encapsulated in spherules.

9. Cosmetic process for depigmenting and/or bleaching human skin, excluding a therapeutic process, characterized in that it consists in applying a composition comprising octopirox to pigmented skin.

## Patentansprüche

1. Verwendung von Octopirox für die Herstellung einer kosmetischen Zusammensetzung oder für die Herstellung

einer dermatologischen Zusammensetzung zur Depigmentierung und/oder Bleichung der menschlichen Haut und/oder zur Entfernung von Pigmentflecken der Haut.

2. Verwendung von Octopirox für die Herstellung einer kosmetischen Zusammensetzung oder für die Herstellung einer dermatologischen Zusammensetzung als Stoff, der die Tyrosinase und/oder die Melaninsynthese hemmt.

3. Nicht-therapeutische Verwendung von Octopirox für die Herstellung einer kosmetischen Zusammensetzung zur Depigmentierung und/oder Bleichung der menschlichen Haut.

4. Verwendung von Octopirox für die Herstellung einer dermatologischen Zusammensetzung zur Depigmentierung und/oder Bleichung der menschlichen Haut.

5. Verwendung nach einem der vorhergehenden Ansprüche, dadurch gekennzeichnet, daß das Octopirox in einer Menge von 0,01 bis 10 % des Gesamtgewichts der Zusammensetzung enthalten ist.

6. Verwendung nach einem der vorhergehenden Ansprüche, dadurch gekennzeichnet, daß das Octopirox in einer Menge von 0,3 bis 3 % des Gesamtgewichts der Zusammensetzung enthalten ist.

7. Verwendung nach einem der vorhergehenden Ansprüche, dadurch gekennzeichnet, daß die Zusammensetzung außerdem mindestens einen Wirkstoff enthält, der unter Keratolytika und/oder abschuppenden Mitteln, entzündungshemmenden Mitteln, beruhigend wirkenden Mitteln und deren Gemischen ausgewählt wird.

8. Verwendung nach dem vorhergehenden Anspruch, dadurch gekennzeichnet, daß das Octopirox und/oder der Wirkstoff in Kügelchen eingekapselt ist.

9. Kosmetisches Verfahren zur Depigmentierung und/oder Bleichung der menschlichen Haut unter Ausschluß eines therapeutischen Verfahrens, dadurch gekennzeichnet, daß auf die pigmentierte Haut eine Zusammensetzung, die Octopirox enthält, aufgetragen wird.

Fig. 1